(19) **Europäisches Patentamt**
**European Patent Office**
**Office européen des brevets**

(11) **EP 2 624 754 B1**

(12) **EUROPEAN PATENT SPECIFICATION**

(45) Date of publication and mention
of the grant of the patent:
**22.08.2018 Bulletin 2018/34**

(21) Application number: **11779503.9**

(22) Date of filing: **03.10.2011**

(51) Int Cl.:
**A61B 5/1455** (2006.01)    **A61B 5/1459** (2006.01)

(86) International application number:
**PCT/US2011/054621**

(87) International publication number:
**WO 2012/047806 (12.04.2012 Gazette 2012/15)**

(54) **SYSTEM, METHOD AND COMPUTER PROGRAM PRODUCT FOR OPTICAL MEASUREMENT OF BLOOD PARAMETERS**

SYSTEM, VERFAHREN UND COMPUTERPROGRAMMPRODUKT ZUR OPTISCHEN MESSUNG VON BLUTPARAMETERN

SYSTÈME, PROCÉDÉ ET PRODUIT PROGRAMME INFORMATIQUE POUR MESURE OPTIQUE DE PARAMÈTRES SANGUINS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2010 US 391219 P**

(43) Date of publication of application:
**14.08.2013 Bulletin 2013/33**

(73) Proprietor: **Edwards Lifesciences Corporation Irvine, CA 92614 (US)**

(72) Inventors:
• **YOUNG, Clayton**
**Irvine**
**CA 92614 (US)**

• **HATIB, Feras**
**Irvine**
**CA 92614 (US)**

(74) Representative: **Eisenführ Speiser**
**Patentanwälte Rechtsanwälte PartGmbB**
**Postfach 31 02 60**
**80102 München (DE)**

(56) References cited:
**US-A- 5 284 137**    **US-A1- 2008 200 780**
**US-A1- 2010 042 004**    **US-B1- 6 430 513**

**Description**

FIELD OF THE INVENTION

This invention relates to a method and a system implementation for determining blood parameters. In particular, the present invention is directed to simultaneous measurement of blood analytes, using optical spectroscopy. The invention is defined by the claims.

BACKGROUND OF THE INVENTION

A standard way to measure blood parameters, such as $SO_2$ (oxygen saturation), $O_2Hb$ (oxyhemoglobin percentage) or tHb (total hemoglobin concentration), in a patient is to direct light into or through the blood, to measure the intensity of the light at either discrete wavelengths or over a substantially continuous spectral range after transmission through or reflection by the blood, and then to calculate the parameter as a function of the measured intensity values.

Many factors reduce the accuracy of known blood parameter monitors that rely on optical measurements as the interrogating method. Beginning with the light source itself, it must be able to produce sufficient light at given wavelengths over a broad wavelength range, and it should do so stably over the life of the instrument.

Observation of transmited light through blood is affected by inconsistencies in optical properties of blood or tissue. When light is directed into blood using a non-invasive device, such as a finger or ear lobe cuff for example, surface variations at the skin/device interface and tissue variations in the vascular bed containing the blood can influence light transmission which ultimately perturbs the measurement. These variations are generally associated with the optical absorption and scattering properties of either tissue or blood.

One source of error that degrades the accuracy of most non-invasive optical monitors is patient motion. Motion artifact leads to a change in the path length of the light through the biological tissue and hence to a variation in the intensity of the detected transmitted or reflected light. The magnitude of measurement noise can be so great that it can render these devices inoperative for long periods of time. The problem is particularly severe in critical healthcare applications, where continuous monitoring is essential, and measurements are in a life-critical application as opposed to ambulatory monitoring.

Another factor that reduces the accuracy of non-invasive optical monitors is skin pigmentation. Many existing optical devices do not take into account the variations in transmitted light caused by varying skin colors, which range from fair through dark brown as the concentration of melanin increases. The peak of melanin's absorption spectrum is at roughly 500 nm, decreasing almost linearly with increasing wavelength. Melanin is present in the epidermis. Thus, in the very high concentrations in dark brown skin, melanin can mask the absorption of hemoglobin in the dermis. Even in less brown skin, the absorption by melanin is superimposed on that of hemoglobin so that any algorithm which uses the shape of the absorption spectrum to produce an estimate needs to compensate for the effect of skin pigmentation.

International Patent Application No WO 00/09004 describes an optical device which is adapted to measure $SO_2$. The device operates by passing light through biological tissue and continuously monitoring the transmitted or reflected output signal using a photodetector. However, one difficulty with the device of the prior art is the fact that the use of a limited number of wavelengths as in WO 00/09004 results in a poor signal-to-noise ratio in the detected signal. This reduces the accuracy of the $SO_2$ determination. Further, this limited-wavelength technique is also more prone to ambient interference, such as from surrounding fluorescent lighting.

One way to reduce the impact of the factors mentioned above is to measure $SO_2$ or other blood parameters invasively. In these applications, light is usually directed into blood by means of catheter-mounted or enclosed optical fibers. The light intensity measured to determine an absorption spectrum for the blood is then usually that of diffusely reflected rather than transmitted light. An advantage to the indwelling approach is using the body itself to isolate the measurement from external lighting sources. Disadvantages of these catheter-based measurements are the need to calibrate against a trustworthy standard and the obvious need to penetrate the body thus resulting in the increased risk of nosocomial infection. A lab test of patient blood using a CO-oximeter, or special apparatus such as a calibration device, must be employed to ensure accuracy. However additional bedside chemical instrumentation is expensive and notoriously difficult to maintain.

Regardless of whether the arrangement used to monitor blood parameters is invasive or non-invasive, there is still the problem of converting the measured light spectrum--which comprises intensity values measured at a plurality of wavelengths--into a single, accurate value, while doing so quickly enough to be useful in a real-time, continuous patient monitoring environment. There is therefore a standing need to improve the accuracy and reliability of blood parameter monitors that use optical measurement technology.

## SUMMARY OF THE INVENTION

The problems of the prior art may be overcome by providing an indwelling system for spectroscopically measuring blood parameters. The system includes a light source and waveguide for transmitting light to the blood, a waveguide for capturing remitted light, a spectrometer decomposing the remitted light into its spectral components and a processor for comparing a morphologically distinct portion of the remitted light to a database of known morphologies. Each of the known morphologies corresponds to a measurement value of at least one parameter, such as oxygen saturation. Advantageously, the determined morphologies can uniquely correspond to two or more blood parameters, allowing simultaneous determination of the two parameters, such as oxyhemoblobin percentage ($O_2Hb$) and total hemoglobin (tHb). One example includes a system for optically measuring parameters in blood and includes a light source, waveguides, spectrometer and a processor. The light source is configured to generate light over a broad spectrum. Generated light from the light source is directed into the blood through a fiber optic waveguide. The remitted light is captured by an adjacent fiber optic waveguide and directed to a spectrometer. The spectral composition of the remitted light is determined by the spectrometer. The processor compares the spectral composition to a database of known morphologies. Each of the known morphologies corresponds to a known value for at least one blood parameter.

In another example each of the known morphologies is defined by a reference matrix of at least three dimensions. And, each of the known morphologies corresponds to the known values for at least two parameters, such as $O_2Hb$ and tHb. In one aspect, the matrix has 3 dimensions of at least 61 x 467 x 101elements. The $1^{st}$ dimension of the matrix represents a spatial wavelength region(s). In another aspect, the $2^{nd}$ dimension of the matrix represents $O_2Hb$ and the $3^{rd}$ dimension can represent tHb. The matrix thus comprises 6161 unique curve elements associated with each relevant combination of O2Hb and tHb.

In another example the light source is a broad spectrum light source in the visible light range, such as a white LED light source. And, the light transmitter and receiver are optical waveguides, such as optical fibers. The optical fibers can be delivered via a catheter into the vasculature of the patient to access the blood.

In yet another example the known morphologies are derived from remitted spectra of 600 nm or less, and more preferably from remitted spectra of 440 nm to 540 nm. Alternatively, or in addition, the known morphologies may be derived from remitted spectra of 715 nm to 800 nm.

In another example the known morphologies are interpolated from known curves at incremental slices of 1% or less for $O_2Hb$ and .1 g/dL or less for tHb.

In another example the return spectrum is filtered and smoothed to suppress spurious information embedded in the signal.

In yet another example the processor is configured to apply a least-squares fit to compare the morphologically distinct portion of the spectral composition to the database of known morphologies. And, the processor can smooth the plurality of known values using a weighted average.

Advantageously, the present invention does not require intermittent calibration during field use, reducing and the number of blood draws and labor intensiveness in routine validation of calibrations. Also, there is no need for installation of a calibration cup at time of manufacture. Also, multiple parameters or analytes, such as O2Hb and tHb, can be measured simultaneously through consultation of morphologically distinct curves.

These and other features and advantages of the present invention will become more readily apparent to those skilled in the art upon consideration of the following detailed description and accompanying drawings, which describe both the preferred and alternative examples, partly not forming part of the invention as defined in the claims.

## BRIEF DESCRIPTION OF THE DRAWINGS

FIG. 1 is a line graph of the different light absorption spectra of blood at different levels of oxygenation over a span of the visible spectrum;
FIG. 2 is a block diagram of the main hardware and software components of a system;

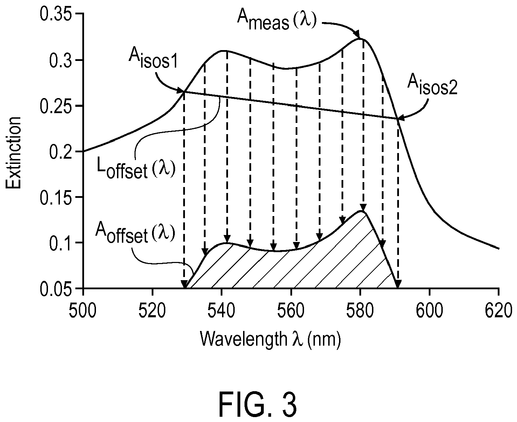

FIG. 3 is a block diagram of a method of normalizing absorption spectra;
FIG. 4 is a line graph of the absorbance of light by oxy and deoxyhemoglobin over the visible and NIR spectrum;
FIG. 5 is a line graph of the backscattered transmission of visible light by oxy and deoxyhemoglobin state;
FIG. 6 is an isolated, morphologically distinct portion of the curve of FIG. 5;
FIG. 7 is a line graph of an interpolation between the curves shown in FIG 6;
FIG. 8 is a line graph of the transmission of visible light by varied levels of Hb concentration within morphologically distinct wavelength ranges;
FIG. 9 is a line graph of an interpolation between the curves of FIG. 8;
FIG. 10 is a schematic illustrating the three-dimensional nature of a reference curve matrix;

FIG. 11 is a line graph of the morphologically distinct relationship of longer light wavelengths absorbed by various levels of oxygenation of Hb;

FIGS. 12-19 are line graphs of experimental data illustrating performance compared to benchmark measurements of $O_2$Hb and tHb; and

FIG. 20 is a line graph of remitted spectra from about 460 nm to 530 nm wavelength using different integration times.

## DETAILED DESCRIPTION OF THE INVENTION

The present invention(s) now will be described more fully hereinafter with reference to specific embodiments of the invention. Indeed, the invention can be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise. The term "comprising" and variations thereof as used herein is used synonymously with the term "including" and variations thereof and are open, non-limiting terms.

A system, method and/or computer program product for measurement of blood analytes of the present invention generally includes a light source for illuminating blood through a range of wavelengths, an optical path to both transmit and receive the light from the source, and a spectrometer (use broadly herein to refer to any device for measuring spectral components) for separating and measuring spectral components of the light and a processing system for determining and using morphologically distinct portions of the resulting absorption, transmission or reflectance spectra to determine the presence of blood analytes or other blood parameters.

Advantageously, several blood analyte readings, such as oxygenated hemoglobin ($O_2$Hb) or total hemoglobin (tHb), may be simultaneously returned by the system using the same measured absorption or transmission spectra without the need for calibration. For example, a three-dimensional reference volume may be built from sets of reference planes characterizing both $O_2$Hb and tHb in an area of the spectra that exhibits changes in curve shape that are distinct for combinations of those two analytes. This reference planar surface is then compared to actual measured values to determine simultaneously $O_2$Hb and tHb. This concept can also be extended to higher dimensions and/or for more or different analyte combinations as long as morphologically distinct portions of the absorption and/or transmission spectra correspond to these analytes.

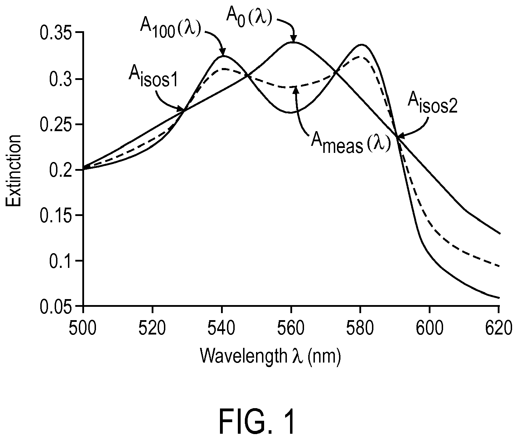

FIG. 1 illustrates several characteristics of light absorption by blood over a range of wavelengths from green to red. In this discussion, $A_x(\lambda)$ represents an absorption spectrum of blood with x % oxygenation (as an example of one analyte) whereas $A_y$ represents an absorption value at wavelength y. In FIG. 1, three spectra are illustrated: $A_0(\lambda)$ and $A_{100}(\lambda)$, representing fully deoxygenated and fully oxygenated blood, respectively, and $A_{meas}(\lambda)$, representing an absorption spectrum that is measured in an actual subject using any invasive or non-invasive technique. For all patients, meas is between 0 and 100. In other words, the actual analyte (such as SaO2, tHb or $O_2$Hb) value for a patient will always be between 0% and 100%. Given an actual measured absorption spectrum $A_{meas}(\lambda)$, the question then becomes what analyte value the spectrum represents. And, the present invention in one embodiment includes determination of not just one, but multiple analytes using this principal.

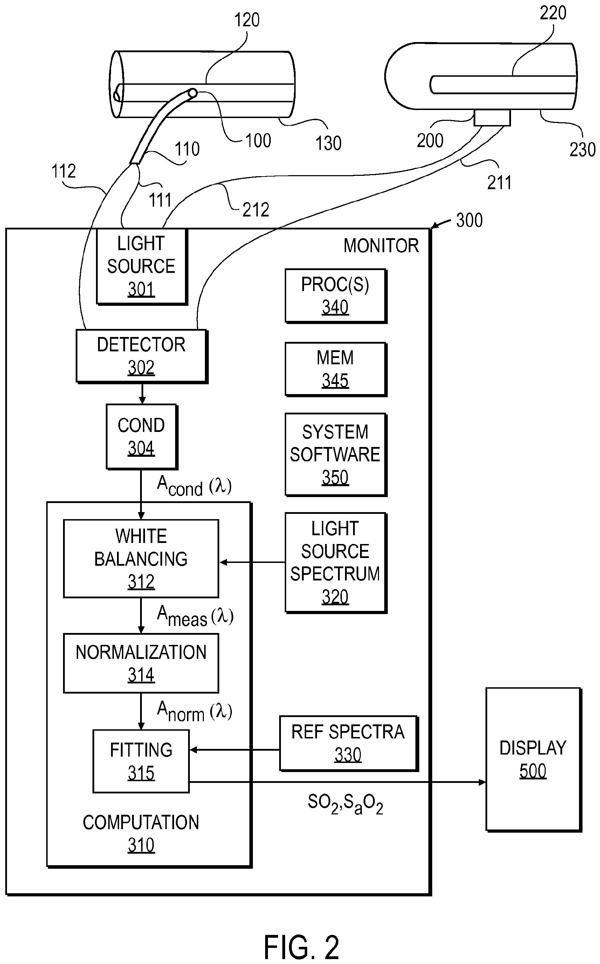

In an example the present system generally includes a monitor 300 connected to a catheter 110 or a finger cuff 200 that deliver light to the patient's blood in an artery, vein or other tissue and return remitted light to the monitor for processing to determine the composition of various analytes in the blood which are displayed on a display monitor 500, as shown in FIG. 2. Notably, the present system could also be used to determine composition in more of a bench-top environment, where a sample of blood is taken from the patient and its spectral properties determined for processing by the monitor 300.

Although some advantages may be had in sensitivity or accuracy of different methods and devices for sensing light transmission or reflection of the blood, embodiments of the present invention is restricted to noninvasive measurements and the invasive options defined here are only exemplary, not forming part of the claimed invention. The light is led to the blood directly and invasively, for example, through one or more optical fibers 111 mounted on or in a catheter 110 to a coupler or lens 100 (which may simply be the end of the transmission fiber), such as is shown in FIG. 2. In this example, the catheter 110 resides within a vessel or other lumen 120 having blood flow within a patient appendage 130.

Or, in an alternative example, measurements may be made indirectly and non-invasively by being conveyed through one or more optic fibers 211, such as a bifurcated fused silica fiber, and then being directed against the skin of a patient's finger 230, or other portion of the body, using a finger cuff 200 or other device in proximity to the skin. In this example, the blood within a vessel 220 is being measured through the intervening skin and not directly as the catheter 110. These non-invasive devices may be placed against the skin at varied body locations but preferably are in locations with prominent blood flow beneath the skin, such as the finger or ear lobe.

Either dedicated optical fibers 112, 212 may be used to capture and convey the remitted light back to the monitor 300, or the transmission fibers 111, 211 may carry the remitted light in duplex mode with additional optical components.

Regardless of the source of the light measurements, the remitted light is decomposed and processed to determine the desired analyte compositions using a combination of hardware, and software. For example, FIG. 2 illustrates hardware and software components of the monitor 300 of an embodiment of the present invention including a light source 301, a detector 302, a conditioner 304, a computation module 310, a processor 340, memory 345 and system software 350.

The system hardware may include one or more processors 340, system memory 345, and system software (such as an operating system) for coordinating and controlling the various hardware devices within the monitor 300, as well as executing the processor-executable code that implements the different software modules described below. Other hardware and software components of a conventional computer can also be included in the monitor 300 as needed.

The source of light 301 is preferably broadband within the visible spectrum and with sufficient spectral energy to allow for adequate discrimination and measurement resolution, at least over the wavelength range that includes the isosbestic wavelengths that lie in the range of 400-600 nm. White light has, by definition, uniform spectral energy within the visible spectrum in the range of 400-600 nm. Incandescent, fluorescent, xenon, deuterium, and tungsten-halogen bulbs may also be used to approximate white light. Greater thermal stability and longer life can usually be obtained at lower cost by using white-light LEDs, however, and for that reason these solid-state devices are preferred.

LEDs also have other advantages for use in embodiments of the present invention. As a semiconductor, they are scalable in high-volume manufacturing. They generate no ultraviolet (UV) light -- long exposure to high intensity UV can produce tissue problems (that is, sunburn). Infrared (IR) light transmission is minimal, contributing to improved thermal stability. And, as a result of the aforementioned lack of UV light, all the optical power emitted by the LED is usable within the wavelength range of interest and, furthermore, no optical filtering is needed to remove unwanted spectral content. LEDs can be turned on and off rapidly, so they can be interleaved to allow a reference measurement.

One problem with many conventional LEDs, however, is that their encapsulant yellows over time, which causes shift to longer wavelengths. Some newer LEDs use a silicone gel, however, as an encapsulant. These LEDs typically retain their original transmission spectrum much better over their exceptionally long normal lifespan, which typically exceeds 80000 hours of operation.

Various types of light-detectors 302 may be used to measure the blood's absorption spectrum. For example, the light-detector may use an array of photodetectors (CMOS), each positioned within the spectrometer and tuned to the wavelength of a respective wavelength transmitted from the LED in the light source 301. Another is an array of changed couple devices (CCD) positioned in the same manner as the CMOS device described above. As mentioned above, though, the preferred light source is a broadband white (or near white) light source. This avoids the need for separate optical transmission fibers (one per wavelength) for separate LED colors and also provides sufficient spectral energy over the wavelength region of interest. It also avoids high part count associated with a multi-LED arrangement, management of aged discoloration of several sources, and load balancing electronics to support multiple sources. In a preferred embodiment of the invention, the detector 302 is a spectrometer that generates the measured spectrum using various optical and opto-electronic components. Signal quality of the remitted spectra captured by the light detector 302 may be improved by varying the intensity level or exposure time.

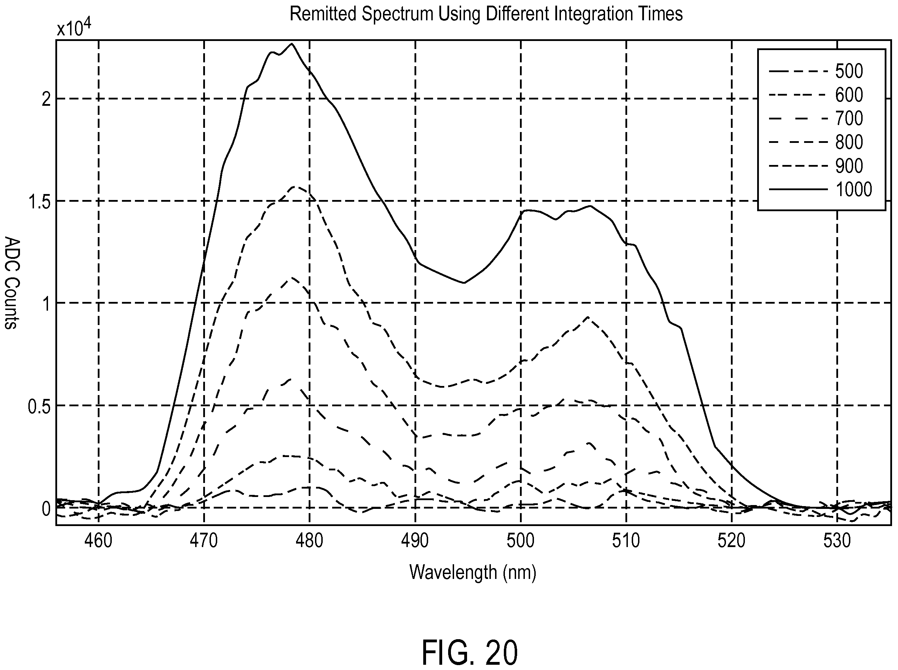

In another sample as shown in FIG. 20, a less sensitive CMOS-based spectrometer with long integration times may be used for acquisition of remitted spectra. Longer integration times can provide improved signal amplitude to resolve the spectral shapes. Shapes in certain instances may also be enhanced with lower integration times. However, the noise is at a fixed intensity so that small shapes at low intensities have fractionally higher amounts of noise. The long integration time signal has the same intensity of noise, but the fractional amount compared to the signal is relatively small.

The signal from the detector 302 can be conditioned using known circuitry 304 before being processed digitally. Such conditioning will normally include various forms of filtering, scaling, analog-to-digital conversion, etc. The result of the conditioning will be a conditioned absorption spectrum $A_{cond}(\lambda)$, or conditioned backscattered transmission $T_{cond}(\lambda)$.

As mentioned above, the spectrum of the light source 301 will not be perfectly white and may have a hue. This will affect the accuracy of the analyte calculations. A "dip" in the measured spectrum might have nothing to do with the blood absorption, for example, but rather with a lower-intensity spectral region in the transmitted light. Different methods may be used to compensate for this deviation from pure "whiteness" in the light source so as to determine the measured absorption spectrum $A_{meas}(\lambda)$.

According to one method for white-balancing, a white-balancing software module 312 calculates $A_{meas}(\lambda)$ according to the formula:

$$A_{meas}(\lambda) = \log_{10} \frac{A_{cond}(\lambda) - D_\lambda}{R_\lambda - D_\lambda}$$

where D is a dark reference intensity at each wavelength $\lambda$ and R is a white reference intensity at each wavelength $\lambda$.

$T_{meas}(\lambda)$ can also be white balanced by the following equation:

$$T_{meas}(\lambda)= f(\ R(\lambda)\text{-}R_t(\lambda))^*T_{cond}(\lambda),$$

where $R(\lambda)$ is the white reference spectrum of the LED determined at the time of LED factory installation. $R_t(\lambda)$ is the white reference spectrum of the LED determined at the time of a routine optical module service interval.

The white and dark reference spectra may be determined using various techniques. For example, in one embodiment, before taking a measurement, the optical sensor (100, 200) is exposed to a standard white reflective surface to give a white reference spectrum. A dark reference spectrum is then also obtained by excluding all excitation light from the optical sensor.

An alternative white-balancing method according to another example takes advantage of the known spectral stability of modern long-life LEDs. Given one or more such LEDs as the light source, in particular, those with silicone encapsulation, the spectrum of the light source can be measured once, in an initial characterization step. The parameters of this characterization (after normalization, as described below) can be stored in a non-volatile medium 320 such as an EPROM chip. This chip, or at least the parameters, can be created or determined once, for example by the LED manufacturer as a factory characterization, such that the parameters can be stored with the LED and can be recalled for later use. No further white measurements would then be needed at all. The values of $A_{cond}(\lambda)$ can then be adjusted according to any known balancing algorithm to account for variations in the spectrum of the white-light LED and thus to form $A_{meas}(\lambda)$.

Another example includes a white-balancing method that interrogates the white LED emission spectrum at a time during field servicing of the instrument. As described in previous examples, the white LED spectrum is captured and stored during final assembly of the optical module and stored in memory. However, due to inevitable manufacturing inconsistencies, the aging process and subsequent change in color hue may be unpredictable. Thus, the most reliable way to adjust the white balance over time is to take a measurement and adjust the backscattered transmission spectra appropriately based on the change between LED spectra at factory install and LED spectra after a specified period of use. In another embodiment, light source 301 and detector 302 are directly coupled using fibers 111 and 112 along with an optical attenuator residing between fibers 111 and 112.

There are several wavelengths--isosbestic wavelengths--at which the light absorption of hemoglobin is independent of the degree of oxygenation. Five such isosbestic wavelengths are visible in FIG. 1, two of which, at wavelengths 522.7 nm and 586.0 nm, are labeled $A_{523}$ and $A_{586}$, respectively. Other isosbestic wavelengths are 505.9, 522, 548.6, and 569.7 nm. These standard values are usually rounded, and are reported slightly differently in some literature, depending on the test methodology used.

Finally, the normalized measured transmittance or absorption spectrum is compared in a fitting software module 315 with a plurality of reference absorption spectra 330 (stored in numerical form in a memory region or non-volatile storage device) as described in further detail below, which may then be displayed on display device 500.

The fitting software module 315 of one embodiment of the present invention is configured to apply a signal processing algorithm to the polychromatic light components of the return spectrum and convert the return spectrum into two or more parameters simultaneously, such as $O_2Hb$ and tHb, by comparing the return spectrum to the reference spectra module 330.

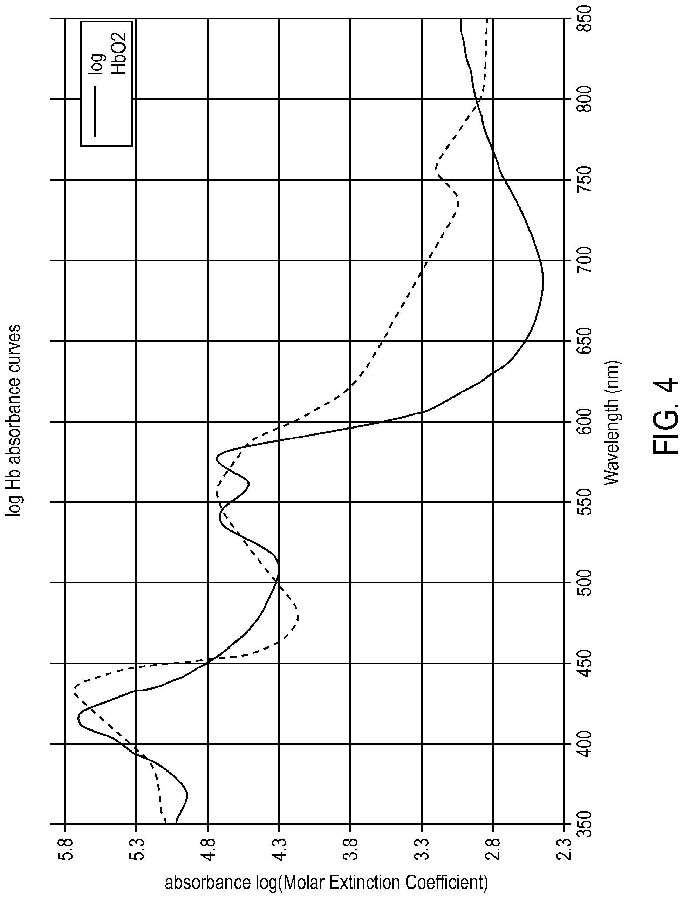

In an example the reference spectra module 330 has a plurality of morphologically distinct curves unique to each combination of $O_2Hb$ and tHb parameters. Similar to blood oxygenation described above, functional hemoglobin has two distinct absorption spectra to visible and near-infrared (NIR) light corresponding to oxygenated and deoxygenated states, as shown in FIG. 4. Absorption is an indication of how much energy is "lost" from the broad spectrum light cast upon the blood by the light source 301. The solid line corresponds to fully saturated Hb near 100% and the dotted line corresponds to fully unsaturated Hb near 0%. Thus, red light near 650 nm to 700 nm is hardly absorbed by the fully saturated Hb, while the majority of energy at 600 nm and shorter wavelengths is heavily absorbed. This is evidenced by the generally brighter red color of fully oxygenated blood which has fully saturated Hb. Meanwhile, unsaturated Hb of deoxygenated venous blood absorbs a more even spectrum throughout the longer wavelengths and has a darker red color.

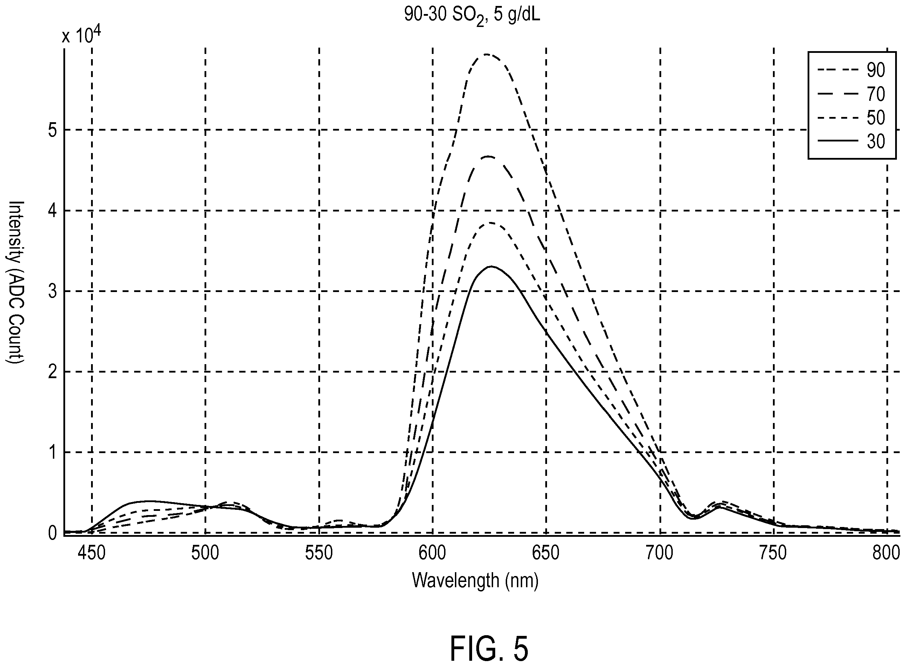

Backscattered transmission spectra provide similar information, although in a different format, because the plot shows the amount of light transmitted through - and not absorbed - by the blood using a white LED, as shown in FIG. 5. The transmission spectra from delivered white LED light in FIG. 5 correspond to oxygenated values between 30% and 90% in intervals of 20%. Measurements were made using a USB4000 spectrometer (Ocean Optics, Dunedin, FL) with a high power white LED source. As the expected inverse of FIG. 4, red light at 600 nm to 700 nm is mostly transmissive, evidencing the generally red color of blood. At 90% oxygenation, red light is highly transmissive compared to adjacent wavelengths, evidencing the bright red color of arterial blood. The venous blood at 30% has a more even transmission relative to adjacent colors - evidencing as a darker red color.

Although the strength of the spectral return signal is very high in the red region of the curve, the wavelengths above 600 nm are not as morphologically distinct for some types of blood parameter measurements. That is, their shapes are similar outside their vertical amplitude and wavelength. The advantage of this characteristic is that the amplitude of these ranges can be easily measured and correlated to oxygenation measurements. However, unlike embodiments of the present invention, some baseline calibration for oxygenation needs to be established to tie amplitude to oxygenation of the blood being sampled.

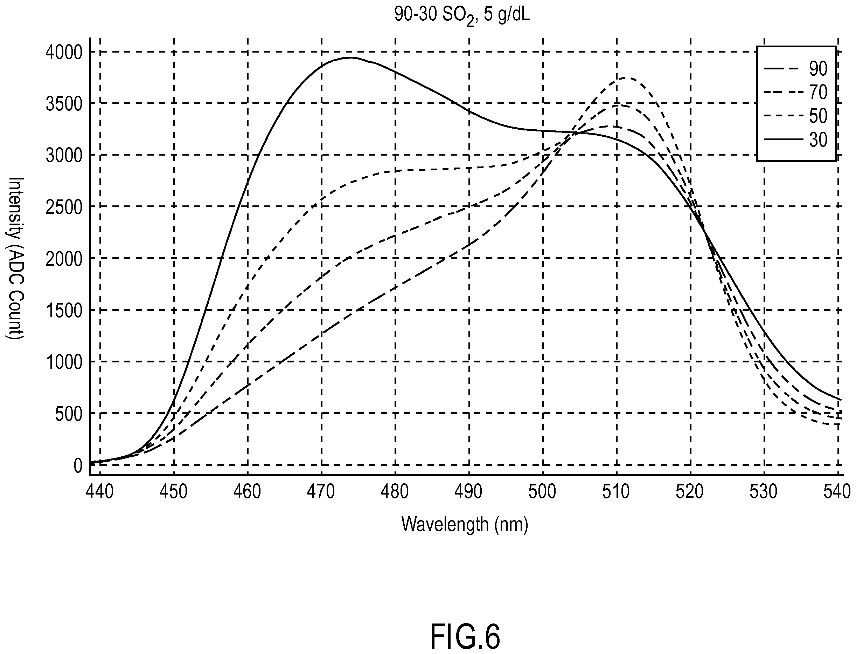

Conversely, the shorter wavelengths of the spectral transmission curve at 600 nm or less are more morphologically distinct, as shown in FIG. 6. To quantify the distinctiveness and to subsequently correlate this to a specific concentration of analyte, a least-squares approach can be used. For each remitted spectra, a comparison is made between the expected spectra in a library/database versus the spectrum currently remitted. The error, or residual, arising from the least-squares approach, can be computed which characterizes the amount of geometric dissimilarity between curves.

Alternatively, instead of quantifying geometric dissimilarity between two states, it is possible to expand the error function to three or more analytes by estimating the presence of three or more analytes at the linear combination of three separate spectra, wherein:

$$S(\lambda) = A_1 x_1(\lambda) + A_2 x_2(\lambda) + A_3 x_3(\lambda) + \ldots + A_n x_n(\lambda)$$

and S is the remitted spectrum, $x_n$ is the spectral signature of the analyte, and $A_n$ is the concentration of the analyte.

FIG. 6 illustrates a scaled version of the sub-600 nm range from the plot shown in FIG. 5. Scaling, filtering and other signal processing techniques, such as by using the isosbestic points as described above, enables more accurate detection and differentiation of the morphology of the curves corresponding to the desired analyte parameter, such as $O_2Hb$ and tHb. This overcomes some of the disadvantage of the strong signal lost when moving away from the greater than 600 nm range or longer than 750 nm. Other methods to improve extraction of morphological features are to transform the spectra using approaches typically found in harmonic analysis or correlation analysis. These methods do not increase the amount of information, but rather rearrange information in other forms that are more easily interpreted. Transforms include, but are not limited to: Fourier transforms, wavelet transforms, non-linearity analysis, higher order statistical moments (skewness, kurtosis, etc.).

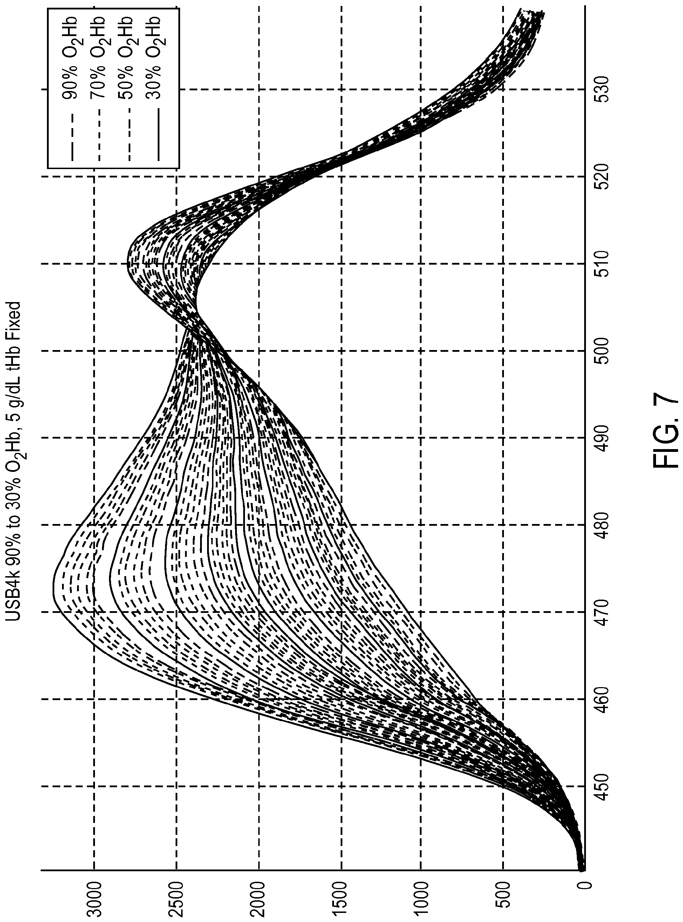

In an example a family of curves is generated through linear and quadratic interpolation, or other means of approximation, between the known, experimentally determined and morphologically distinct curves. For example, FIG. 7 illustrates linear interpolation at 1% $O_2Hb$ increments between the four experimentally determined curves at 30%, 50%, 70% and 90% $O_2Hb$ levels of the curve in FIG. 6. This results in a generation of 61 curves for FIG. 7. These curves were determined by flowing blood through a test chamber and assessing the state of flowing blood using an IL682 CO-oximeter (Instrumentation Laboratory, Bedford, MA) which took discrete samples. The resolution of this family of curves could also be improved by reducing the increment for interpolation.

Another example includes extending the above-described process into additional dimensions for other analytes. Advantageously, when sufficient overlap of morphologically distinct regions of the remitted spectra exists, two or more analytes (or other blood parameters) can be measured simultaneously. For example, the sub-600 nm range of the above-illustrated $O_2Hb$ curve also exhibits morphological distinctiveness for variations in total hemoglobin concentration (tHb). This is likely attributable to the increased bulk amount of phospholipid scatterers in solution that is the packaging for each individual red blood cell. These changes to the concentration of blood change the bulk absorbance and scattering properties that subsequently change the remitted spectra. Other analytes of interest that may be measured by selected embodiments of the present invention include carboxyhemoglobin (COHb) and methemoglobin (MetHb).

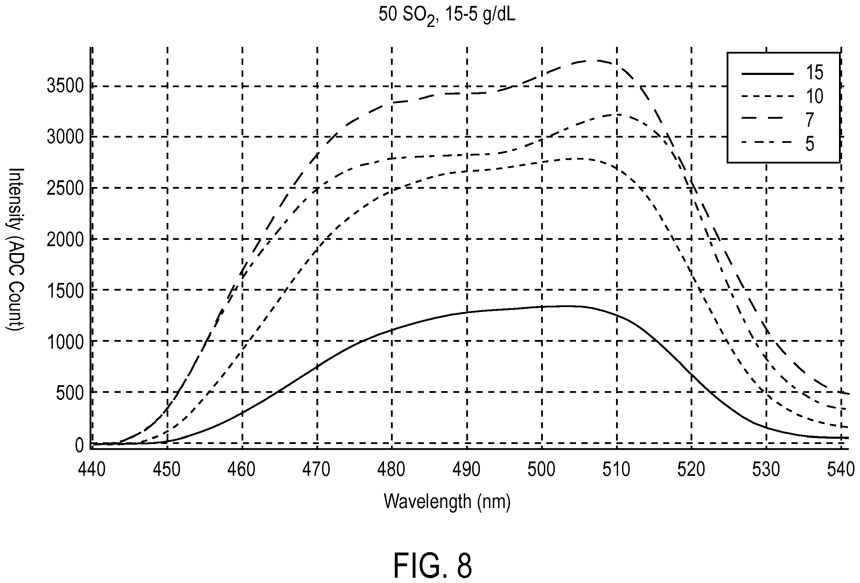

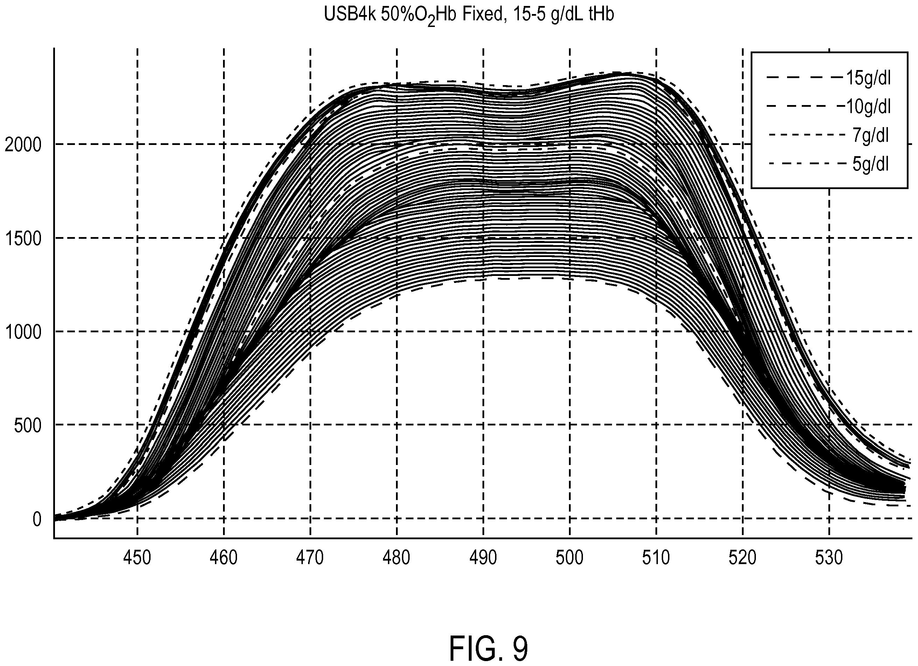

FIG. 8 shows a 50% $O_2Hb$ curve's morphological changes between 5 g/dL, 7 g/dL, 10 g/dL and 15 g/dL. Similar to FIG.7, this curve can be interpolated between those experimentally determined concentrations in increments of .1 g/dL to yield the curve shown in FIG. 9. FIG. 9 is just one "slice" of a three-dimensional curve that maps the morphology of the two analytes - $O_2Hb$ and tHb - for the remitted spectral ranges in the sub-600 nm region.

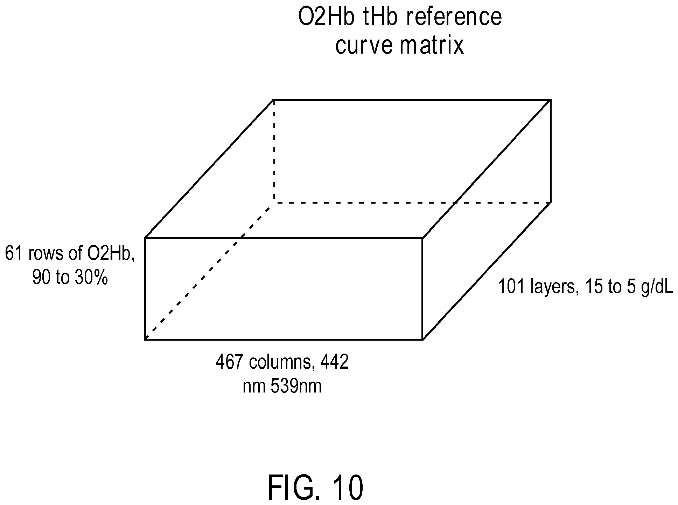

In other words, a full set of curves can be generated by interpolation in an oximetry-wise and hemoglobin-wise directions to yield a 3-dimensional matrix characterizing the morphologically distinct curves unique to each combined $O_2Hb$/tHb measurement from (in the illustrated embodiment) 30% to 90% levels of $O_2Hb$ between 5 g/dL through 15 g/dL of tHb. Interpolating stepwise at 1% for $O_2Hb$ and .1 g/dL for tHb yields 6161 unique curves. In a preferred embodiment, each of the curves is characterized by 467 points between 440 nm and 540 nm to generate a reference matrix S with dimensions of 61 x 467 x 101 as conceptually illustrated by FIG. 10. This matrix is used to populate the reference spectra module 330, for example, of FIG. 2 of an embodiment of the present invention.

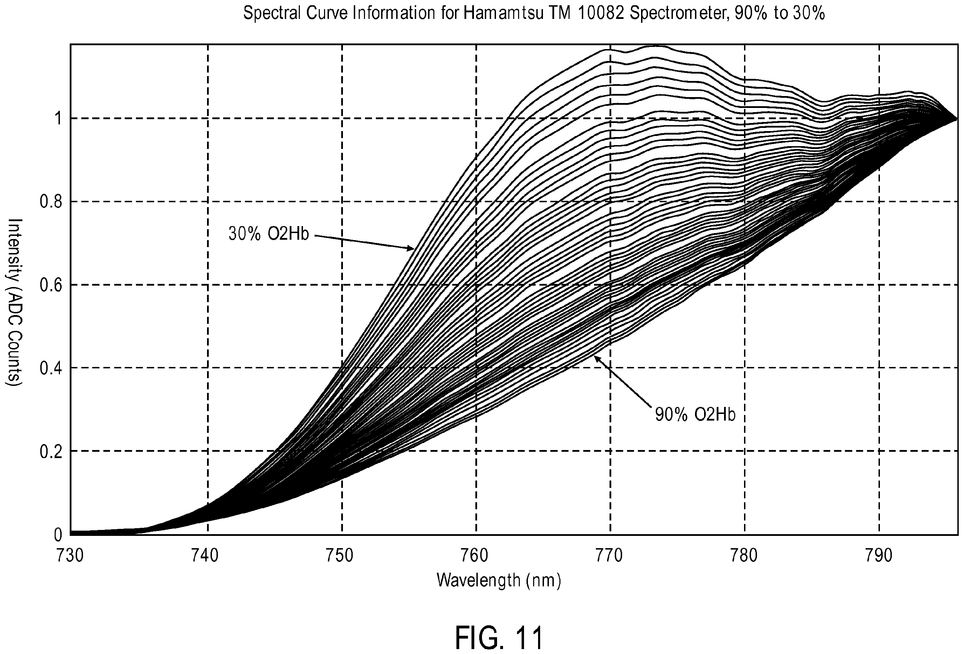

In addition, it should be noted that distinct morphology isn't limited to the sub-600 nm or blue-green 440 nm to 540 nm remitted spectra ranges of the above-described embodiments. For example, as shown in FIG. 11, another example includes longer wavelength ranges, such as 730 nm and 796 nm, where deoxyhemoglobin and oxyhemoglobin are also morphologically distinct. These regions can be extended to 2, 3 or more parameters each with the addition of at least one dimension to the reference spectra matrix as long as the remitted spectra are sufficiently morphologically distinct to correlate to known combinations of the parameter measurements. Also, other wavelength ranges may be evaluated in parallel to enhance error detection of the same parameter, such as the error caused by wall artifact. Error would be reduced by bringing the "cube" of the matrix into higher dimensions of an n-dimensional error space.

Some other characteristics of "distinctiveness", which could also be described as "features" or "figures of merit", could be employed for building, and later correlation to, libraries or databases of known blood parameters. One example is power over a wavelength range which can be determined from area under the curve. Another example is linearity over a wavelength range. See, for example, the 90% curve in FIG. 7 which can be compared to a straight line and its deviation quantified. Relative power between wavelength ranges could also be employed, such as by determining ratios of areas between parts of the spectrum. Further each of these features could be combined with the error-fit approach described above for improved results.

Fitting module 315, in another example is configured to process an incoming spectrum from detector 302 passed through conditioner 304 acquired over a given period of time, such as from 4 milliseconds to several hundred milliseconds. Regardless of length, this return spectrum can be characterized as a vector $r$:

$$r = [r1\ r2\ r3\ \dots\ rn],\ \text{where, in this embodiment, n} - 1, 2, 3, \dots\ 467$$

The rows of the reference spectrum matrix are $i$ and the columns are $j$, while the layers are $k$. Thus, for each remitted spectrum acquired, a two-dimensional error function is defined by:

$$error(i,k) = \sum_{n=1}^{467} [S(i,n,k) - rn]^2$$

The smallest value of the error matrix at coordinate i,k of the error plane is linked to the corresponding $O_2Hb$, tHb curve at this particular point. Thus, the minimum error curve is the best fit for this coordinate pair and is the most likely estimate of $O_2Hb$ and tHb.

For the tHb measurement, the fitting module 315 (or alternatively, or in combination, the conditioning module 304) is configured to perform a running weighted average to smooth the measurement yielded by least-squares fit, such as by using the following equation:

$$tHb_{reported}(i) = (.1)tHb_{calculated}(i) + (.9)tHb_{reported}(i\text{-}i)$$

where i is the present measurement and i-1 is the previous measurement. The weighted average could be employed over other wavelength ranges so that measurement failure in one range does not necessarily cause a significant error in the overall parameter reading.

The above-described method for fitting module 315 is a least-squares method. But, other methods are also possible and can be used based on the morphology of the curves being employed as the data for the reference spectra module 330. Also, multivariate or principal component analysis can be used to compare the signal fidelity and subsequent data output associated with different wavelength regimes.

As a further refinement, other examples may address reducing computational overhead. To reduce the computational need, an algorithm can seek out a smaller number of wavelengths to determine the blood parameters, such as analyte concentrations. If the database or library is sufficiently dense, the algorithm can be trained to differentiate between measurement values using a smaller number of wavelengths.

Although the light source 301 preferably generates white light, the method of computing the oxygenation value by evaluating a cost function of the remitted absorption spectrum relative to the reference spectra 330 could also be used in implementations that transmit discrete wavelengths of light, for example from an array of single-wavelength LEDs, as long as enough wavelengths are included to allow for compilation of a reasonable representation of the remitted spectrum. It is also helpful at low signal levels if at least two of the wavelengths are isosbestic such that they can be used in the spectral normalization procedure.

The method and apparatus for determining one or more blood parameters using remitted spectral light as set forth in FIG. 2 may be embodied by a computer program product. The computer program product includes a computer-readable storage medium, such as the non-volatile storage medium, and computer-readable program code portions, such as a series of computer instructions, embodied in the computer-readable storage medium. Typically, the computer program is stored by a memory device, such as memory 345 and executed by an associated processing element, such as processor 340.

In this regard, FIG. 2 is a flowchart of a method, apparatus and program product according to exemplary embodiments of the invention. It will be understood that each step of the flowchart, and combinations of the steps in the flowchart, can be implemented by computer program instructions. These computer program instructions may be loaded onto a computer or other programmable apparatus to produce a machine, such that the instructions which execute on the computer or other programmable apparatus create means for implementing the functions specified in the flowchart step(s). These computer program instructions may also be stored in a computer-readable memory that can direct a computer or other programmable apparatus to function in a particular manner, such that the instructions stored in the computer-readable memory produce an article of manufacture including instruction means which implement the function specified in the flowchart step(s). The computer program instructions may also be loaded onto a computer or other programmable apparatus to cause a series of operational steps to be performed on the computer or other programmable apparatus to produce a computer implemented process such that the instructions which execute on the computer or other programmable apparatus provide steps for implementing the functions specified in the flowchart step(s).

Accordingly, steps of the flowchart support combinations of means for performing the specified functions, combinations of steps for performing the specified functions and program instruction means for performing the specified functions. It will also be understood that each step of the flowchart, and combinations of steps in the flowchart, can be implemented by special purpose hardware-based computer systems which perform the specified functions or steps, or combinations of special purpose hardware and computer instructions.

EXPERIMENTAL

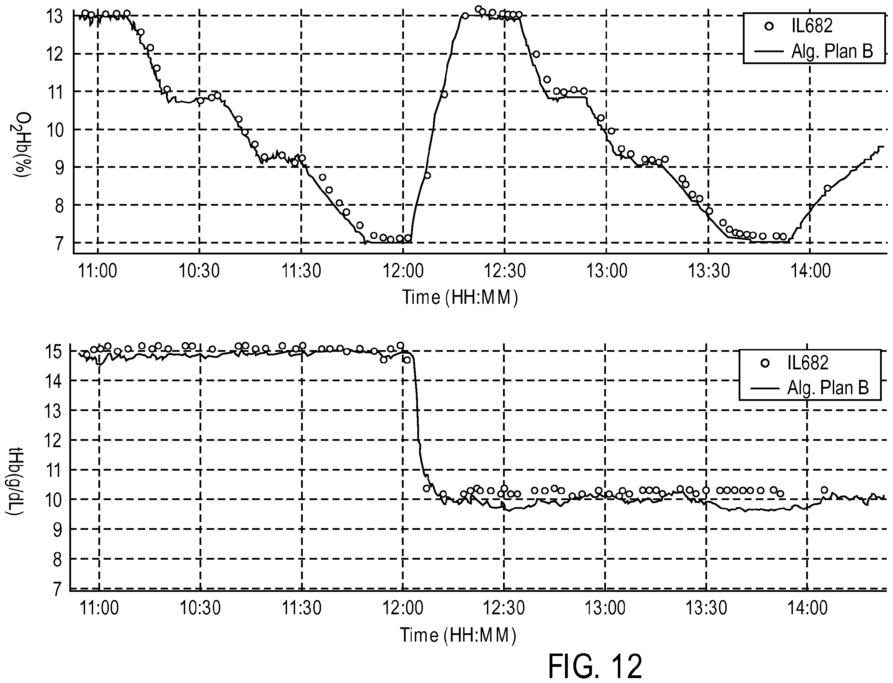

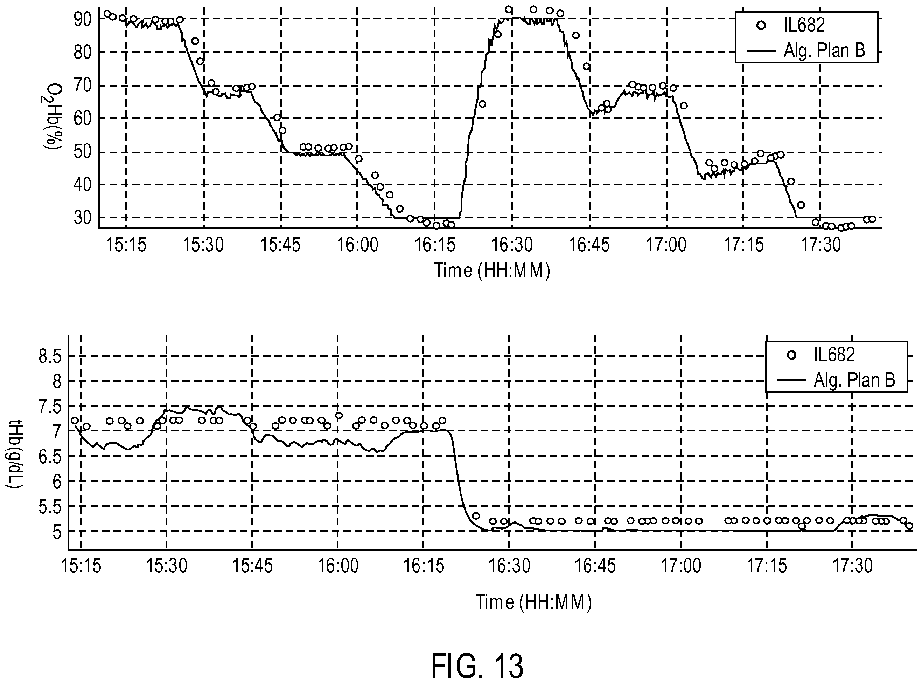

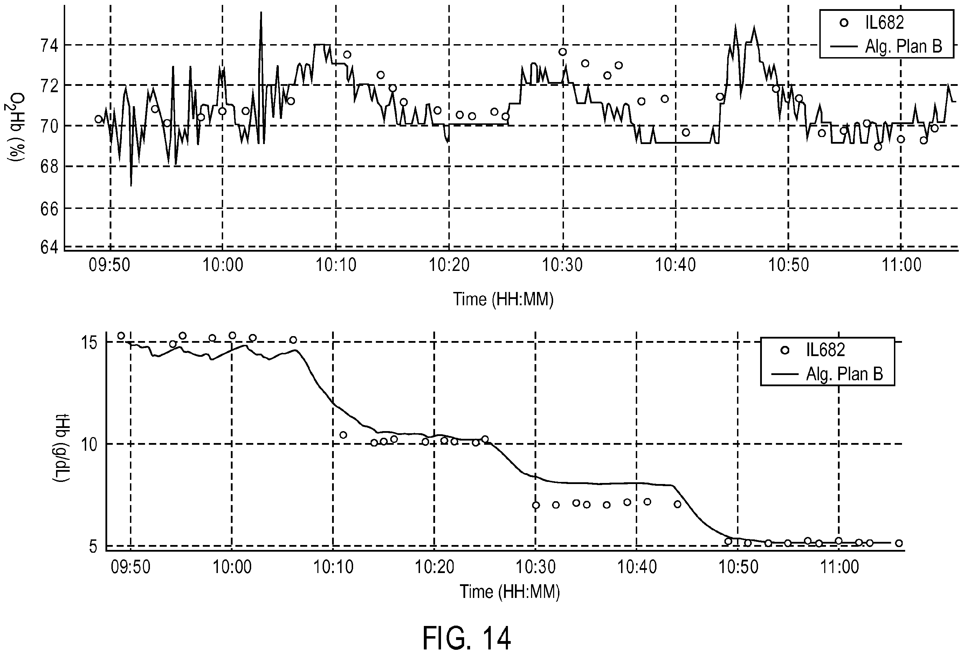

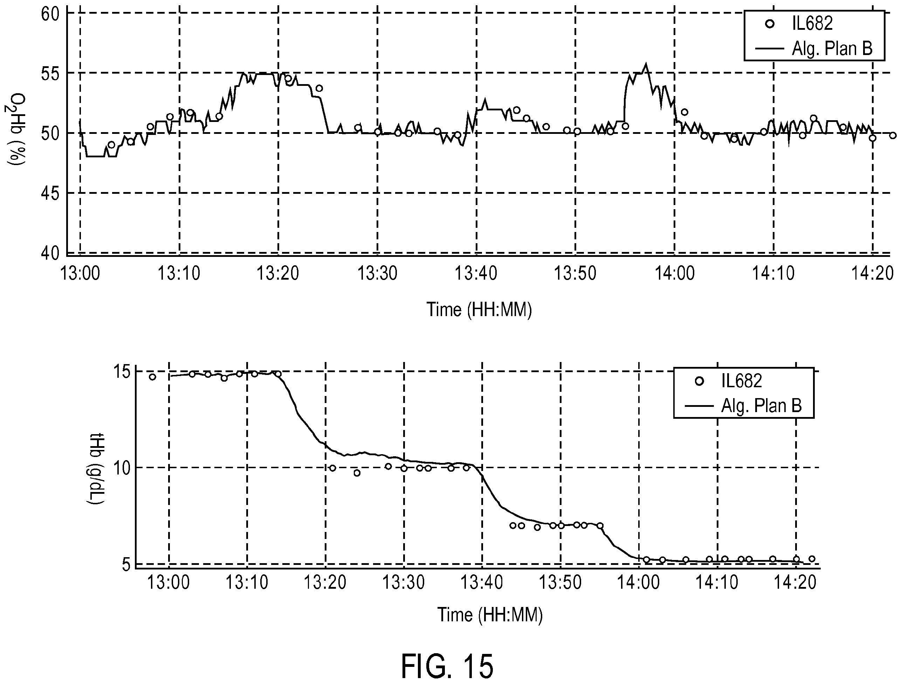

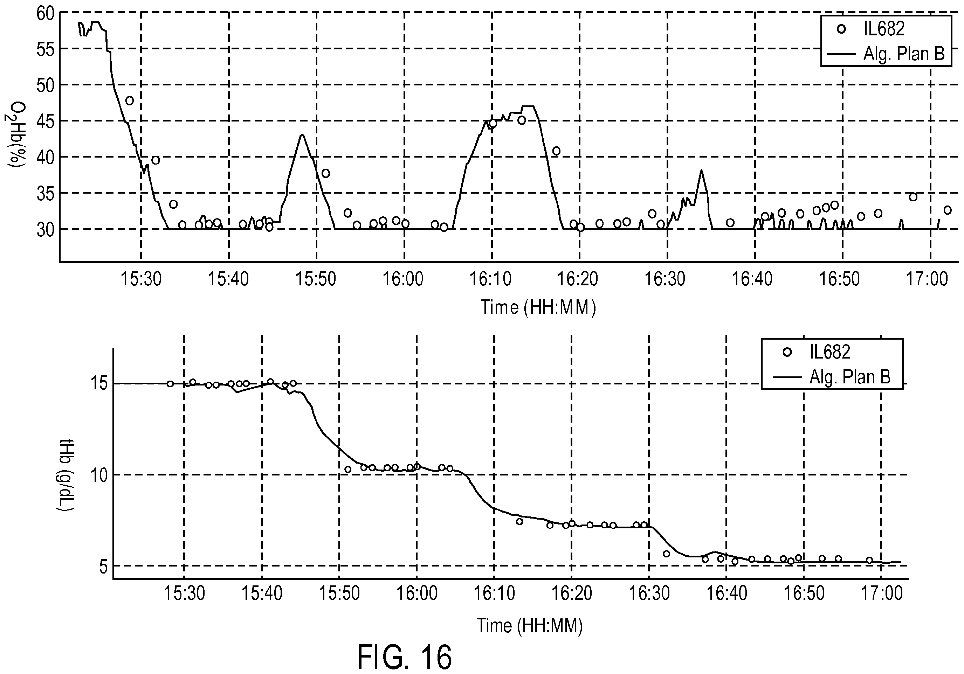

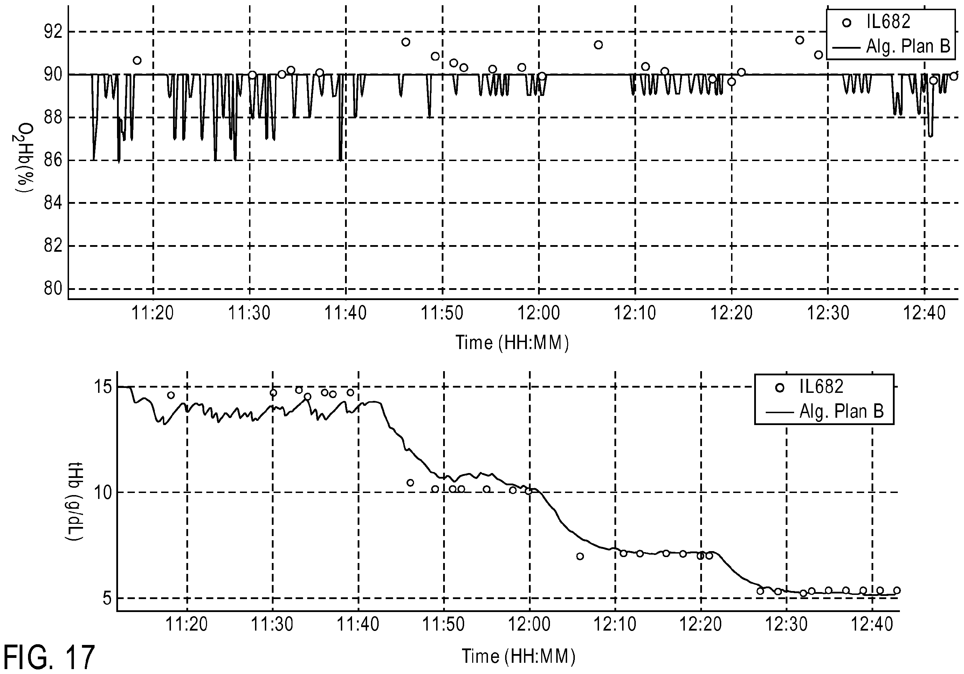

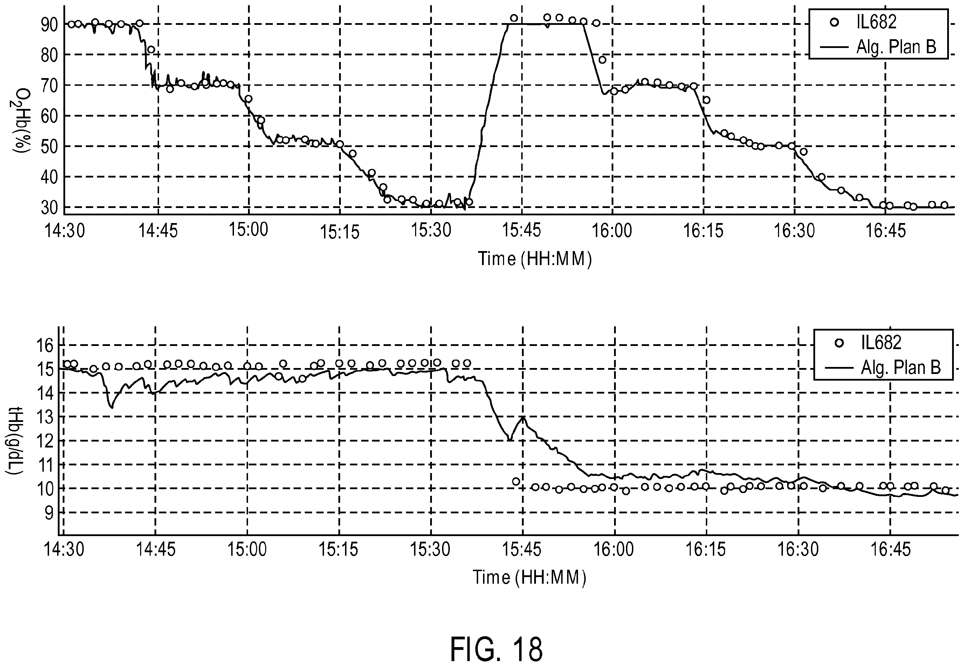

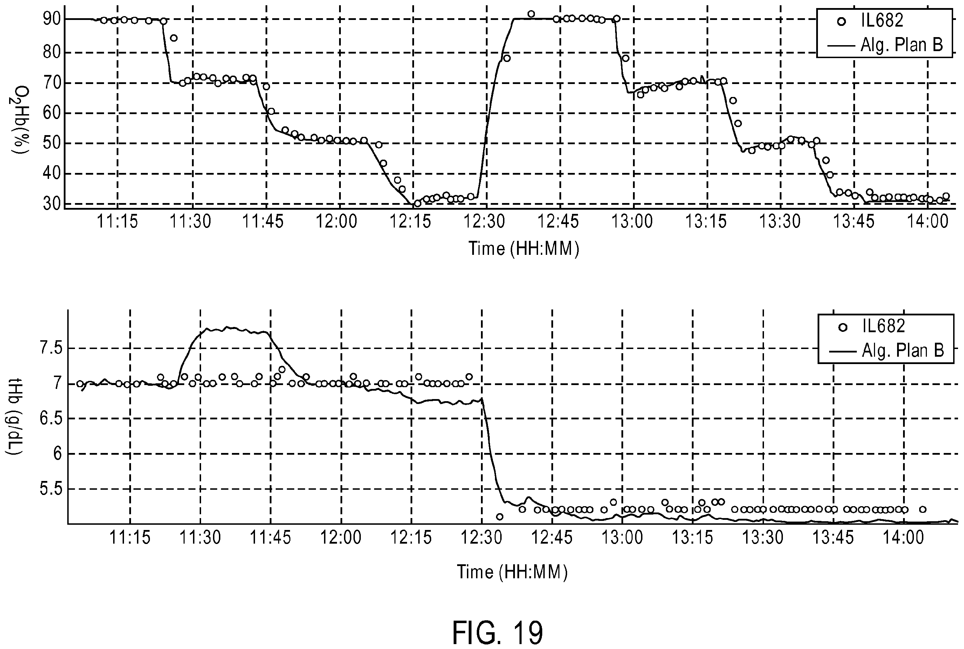

The present invention will now be described with specific reference to various examples. The following examples are not intended to be limiting of the invention and are rather provided as exemplary embodiments. FIGS. 12-19 show the results of a validation study of the embodiment disclosed in FIG. 10 for simultaneous measurement of both $O_2Hb$ and tHb. The same CO-Oximeter (IL682) used to take the training data was used for validation. The dots on the FIGS. 12-19 are the results of the CO-Oximeter while the lines are the algorithm output for either $O_2Hb$ or tHb evaluated retrospectively against the reference curves. Notably, the curves are a good approximation of the values measured by the CO-Oximeter.

Advantageously, the present invention does not require intermittent calibration during field use, reducing healthcare worker labor and blood draws. Also, there is no need for installation of a calibration device at time of manufacture. Also, multiple parameters or analytes, such as O2Hb and tHb can be measured simultaneously through consultation of morphologically distinct curves.

Many modifications and other embodiments of the invention set forth herein will come to mind to one skilled in the art to which this invention pertains having the benefit of the teachings presented in the foregoing description. For example, other wavelength ranges could be correlated in parallel for any particular parameter measurement to reduce the impact of wall artifact. Also, the 440 nm to 540 nm range could be extended to greater ranges to enhance the morphological distinctiveness of the reference curves.

Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

**Claims**

1. A system for optically measuring parameters in blood, the system comprising:

   a light source (301) configured to generate light having a range of wavelengths;
   a light transmitter (111; 211) configured to direct the generated light from the light source (301) into the blood;
   a light remitter (112; 212) configured to receive and direct light remitted from the blood;
   a detector (302) configured to receive the remitted light from the light remitter (112; 212) and determine a spectral composition of the remitted light; and
   a processor (340) configured to compare the spectral composition to a database of known morphologies, wherein the database of known morphologies is defined by a reference matrix of at least three dimensions and each of the known morphologies corresponds to known values for at least two parameters and wherein the known morphologies are derived from a predetermined range of wavelengths,
**characterized in that** the processor (340) is configured to determine a morphology of at least one morphologically distinct portion of the spectral composition, wherein the morphologically distinct portion corresponds to the predetermined range of wavelengths and wherein the determined morphology corresponds to the at least two blood parameters, and the processor (340) is configured to compare the determined morphology to the database of known morphologies to determine the values for the at least two blood parameters.

2. The system of claim 1, wherein the three-dimensional matrix has dimensions of at least 61 x 467 x 101.

3. The system of claim 2, wherein the 467 slices of the matrix represent points between 440 nm and 540 nm.

4. The system of claim 3, wherein the 61 slices of the matrix represent one of $O_2Hb$ or tHb.

5. The system of claim 1, wherein the matrix includes at least 6161 unique curves.

6. The system of claim 1, wherein the light transmitter (111; 211) is a first optical waveguide.

7. The system of claim 6, wherein the light remitter (112; 212) is a second optical waveguide.

8. The system of claim 1, wherein the known morphologies correspond to unique measurements of $O_2Hb$ and tHb.

9. The system of claim 1, wherein the processor (340) is configured to apply a least-squares fit to compare the determined morphology to the database of known morphologies.

10. The system of claim 1, wherein the processor (340) is further configured to report a plurality of the known values for display.

11. The system of claim 10, wherein the processor (340) is further configured to smooth the plurality of known values.

12. A method of optically measuring parameters in blood in a non-invasive manner, the method comprising:

generating light having a range of wavelengths;
directing the light into blood;
receiving remitted light from the blood;
determining a spectral composition of the remitted light; and
comparing the spectral composition to a database of known morphologies,

wherein the database of known morphologies is defined by a reference matrix of at least three dimensions and each of the known morphologies corresponds to known values for at least two parameters and wherein the known morphologies are derived from a predetermined range of wavelengths,
**characterized in that** the method further comprises:
determining a morphology of at least one morphologically distinct portion of the spectral composition, wherein the morphologically distinct portion corresponds to the predetermined range of wavelengths and wherein the determined morphology corresponds to the at least two blood parameters, wherein comparing includes comparing the determined morphology to the database of known morphologies to simultaneously determine the values for the at least two blood parameters.

13. The method of claim 12, wherein comparing includes comparing to the three dimensional matrix with dimensions of at least 61 x 467 x 101.

14. The method of claim 13, wherein comparing includes comparing 467 slices of the matrix between 440 nm and 540 nm.

15. The method of claim 12, wherein comparing includes applying a least-squares fit to compare the determined morphology to the database of known morphologies.

**Patentansprüche**

1. System zum optischen Messen von Parametern in Blut, wobei das System umfasst:

   eine Lichtquelle (301), die dafür ausgelegt ist, Licht zu erzeugen, das einen Bereich von Wellenlängen aufweist;
   einen Lichttransmitter (111; 211), der dafür ausgelegt ist, das erzeugte Licht von der Lichtquelle (301) in das Blut zu richten;
   einen Lichtremitter (112; 212), der dafür ausgelegt ist, das von dem Blut remittierte Licht zu empfangen und zu richten;
   einen Detektor (302), der dafür ausgelegt ist, das von dem Lichtremitter (112, 212) remittierte Licht zu empfangen und eine Spektralzusammensetzung des remittierten Lichts zu bestimmen; und
   einen Prozessor (340), der dafür ausgelegt ist, die Spektralzusammensetzung mit einer Datenbank bekannter Morphologien zu vergleichen, wobei die Datenbank bekannter Morphologien durch eine Referenzmatrix von zumindest drei Dimensionen definiert ist und eine jede der bekannten Morphologien bekannten Werten für zumindest zwei Parameter entspricht und wobei die bekannten Morphologien von einem vorbestimmten Bereich von Wellenlängen abgeleitet werden,
   **dadurch gekennzeichnet, dass** der Prozessor (340) dafür ausgelegt ist, eine Morphologie von zumindest einem morphologisch distinkten Abschnitt der Spektralzusammensetzung zu bestimmen, wobei der morphologisch distinkte Abschnitt dem vorbestimmten Bereich von Wellenlängen entspricht und wobei die bestimmte Morphologie den zumindest zwei Blutparametern entspricht, und dass der Prozessor (340) dafür ausgelegt ist, die bestimmte Morphologie mit der Datenbank bekannter Morphologien zu vergleichen, um die Werte der zumindest zwei Blutparameter zu bestimmen.

2. System nach Anspruch 1, wobei die dreidimensionale Matrix Abmessungen von zumindest 61 x 467 x 101 aufweist.

3. System nach Anspruch 2, wobei die 467 Schichten der Matrix Punkte zwischen 440 nm und 540 nm darstellen.

4. System nach Anspruch 3, wobei die 61 Schichten der Matrix $O_2Hb$ bzw. tHb darstellen.

5. System nach Anspruch 1, wobei die Matrix zumindest 6161 eindeutige Kurven enthält.

6. System nach Anspruch 1, wobei es sich bei dem Lichttransmitter (111; 211) um einen ersten Lichtwellenleiter handelt.

7. System nach Anspruch 6, wobei es sich bei dem Lichtremitter (112; 212) um einen zweiten Lichtwellenleiter handelt.

8. System nach Anspruch 1, wobei die bekannten Morphologien eindeutigen Messungen von $O_2Hb$ und tHb entsprechen.

9. System nach Anspruch 1, wobei der Prozessor (340) dafür ausgelegt ist, eine Anpassung nach der Methode der kleinsten Fehlerquadrate vorzunehmen, um die bestimmte Morphologie mit der Datenbank bekannter Morphologien zu vergleichen.

10. System nach Anspruch 1, wobei der Prozessor (340) ferner dafür ausgelegt ist, eine Mehrzahl von bekannten Werten für die Anzeige zu melden.

11. System nach Anspruch 10, wobei der Prozessor (340) ferner dafür ausgelegt ist, die Mehrzahl von bekannten Werten zu glätten.

12. Verfahren zum auf nichtinvasive Weise erfolgenden, optischen Messen von Parametern in Blut, wobei das Verfahren umfasst, dass

   Licht erzeugt wird, das einen Bereich von Wellenlängen aufweist;
   das Licht in Blut gelenkt wird;
   aus dem Blut remittiertes Licht empfangen wird;
   eine Spektralzusammensetzung des remittierten Lichts bestimmt wird; und
   die Spektralzusammensetzung mit einer Datenbank bekannter Morphologien verglichen wird, wobei die Datenbank bekannter Morphologien durch eine Referenzmatrix von zumindest drei Dimensionen definiert ist und eine jede der bekannten Morphologien bekannten Werten für zumindest zwei Parameter entspricht und wobei die bekannten Morphologien von einem vorbestimmten Bereich von Wellenlängen abgeleitet werden, **dadurch gekennzeichnet, dass** das Verfahren ferner umfasst, dass:

eine Morphologie von zumindest einem morphologisch distinkten Abschnitt der Spektralzusammensetzung bestimmt wird, wobei der morphologisch distinkte Abschnitt dem vorbestimmten Bereich von Wellenlängen entspricht und wobei die bestimmte Morphologie den zumindest zwei Blutparametern entspricht, wobei das Vergleichen umfasst, dass die bestimmte Morphologie mit der Datenbank bekannter Morphologien verglichen wird, um gleichzeitig die Werte der zumindest zwei Blutparameter zu bestimmen.

**13.** Verfahren nach Anspruch 12, wobei das Vergleichen umfasst, dass mit der dreidimensionalen Matrix mit den Abmessungen von zumindest 61 x 467 x 101 verglichen wird.

**14.** Verfahren nach Anspruch 13, wobei das Vergleichen umfasst, dass 467 Schichten der Matrix zwischen 440 nm und 540 nm verglichen werden.

**15.** Verfahren nach Anspruch 12, wobei das Vergleichen umfasst, dass eine Anpassung nach der Methode der kleinsten Fehlerquadrate vorgenommen wird, um die bestimmte Morphologie mit der Datenbank bekannter Morphologien zu vergleichen.

**Revendications**

**1.** Système destiné à mesurer optiquement des paramètres dans du sang, lequel système comprend:

une source lumineuse (301) qui est conçue pour produire de la lumière présentant une plage donnée de longueurs d'onde;
un émetteur de lumière (111; 211) qui est conçu pour diriger la lumière produite depuis la source lumineuse (301) jusque dans le sang;
un réémetteur de lumière (112; 212) qui est conçu pour recevoir et diriger la lumière réémise à partir du sang ;
un détecteur (302) qui est conçu pour recevoir la lumière réémise par le réémetteur de lumière (112; 212) et pour déterminer une composition spectrale de la lumière réémise; et
un processeur (340) qui est conçu pour comparer la composition spectrale avec une banque de données de morphologies connues, la banque de données de morphologies connues étant définie par une matrice de référence d'au moins trois dimensions et chacune des morphologies connues correspondant à des valeurs connues pour au moins deux paramètres et lesdites morphologies connues étant déduites d'une plage prédéfinie de longueurs d'onde,
**caractérisé en ce que** le processeur (340) est conçu pour déterminer une morphologie d'au moins une partie morphologiquement distincte de la composition spectrale, la partie morphologiquement distincte correspondant à la plage prédéterminée de longueurs d'onde et la morphologie déterminée correspondant auxdits au moins deux paramètres sanguins, et **en ce que** le processeur (340) est conçu pour comparer la morphologie déterminée avec la banque de données de morphologies connues afin de déterminer les valeurs desdits au moins deux paramètres sanguins.

**2.** Système selon la revendication 1, dans lequel la matrice tridimensionnelle présente des dimensions d'au moins 61 x 467 x 101.

**3.** Système selon la revendication 2, dans lequel les 467 couches de la matrice représentent des points compris entre 440 nm et 540 nm.

**4.** Système selon la revendication 3, dans lequel les 61 couches de la matrice représentent $O_2Hb$ ou tHb.

**5.** Système selon la revendication 1, dans lequel la matrice contient 6161 courbes uniques.

**6.** Système selon la revendication 1, dans lequel l'émetteur de lumière (111; 211) est un premier guide d'ondes optiques.

**7.** Système selon la revendication 6, dans lequel le réémetteur de lumière (112; 212) est un deuxième guide d'ondes optiques.

**8.** Système selon la revendication 1, dans lequel les morphologies connues correspondent à des mesures uniques

de O$_2$Hb et tHb.

**9.** Système selon la revendication 1, dans lequel le processeur (340) est conçu pour effectuer une adaptation selon la méthode des moindres carrés des erreurs afin de comparer la morphologie déterminée avec la banque de données de morphologies connues.

**10.** Système selon la revendication 1, dans lequel le processeur (340) est en outre conçu pour signaler une pluralité de données connues pour l'affichage.

**11.** Système selon la revendication 10, dans lequel le processeur (340) est en outre conçu pour lisser la pluralité de données connues.

**12.** Procédé destiné à mesurer optiquement de manière non invasive des paramètres dans du sang, ledit procédé comprenant le fait de:

produire une lumière présentant une plage donnée de longueurs d'onde;
diriger la lumière dans du sang;
recevoir de la lumière réémise à partir du sang;
déterminer une composition spectrale de la lumière réémise; et
comparer la composition spectrale avec une banque de données de morphologies connues, la banque de données de morphologies connues étant définie par une matrice de référence d'au moins trois dimensions et chacune des morphologies connues correspondant à des valeurs connues pour au moins deux paramètres et lesdites morphologies connues étant déduites d'une plage prédéfinie de longueurs d'onde,
**caractérisé en ce que** ledit procédé comprend en outre le fait de:
déterminer une morphologie d'au moins une partie morphologiquement distincte de la composition spectrale, la partie morphologiquement distincte correspondant à la plage prédéterminée de longueurs d'onde et la morphologie déterminée correspondant auxdits au moins deux paramètres sanguins, la comparaison comprenant le fait de comparer la morphologie déterminée avec la banque de données de morphologies connues afin de déterminer simultanément les valeurs desdits au moins deux paramètres sanguins.

**13.** Procédé selon la revendication 12, lors duquel la comparaison comprend le fait d'effectuer la comparaison avec la matrice tridimensionnelle présentant les dimensions d'au moins 61 x 467 x 101.

**14.** Procédé selon la revendication 13, lors duquel la comparaison comprend le fait de comparer 467 couches de la matrice comprises entre 440 nm et 540 nm.

**15.** Procédé selon la revendication 12, lors duquel la comparaison comprend le fait d'effectuer une adaptation selon la méthode des moindres carrés des erreurs afin de comparer la morphologie déterminée avec la banque de données de morphologies connues.

FIG. 1

FIG. 2

FIG. 3

FIG. 4

90-30 SO$_2$, 5 g/dL

FIG. 5

EP 2 624 754 B1

FIG.6

USB4k 90% to 30% $O_2Hb$, 5 g/dL tHb Fixed

Legend:
- 90% $O_2Hb$
- 70% $O_2Hb$
- 50% $O_2Hb$
- 30% $O_2Hb$

FIG. 7

EP 2 624 754 B1

**FIG. 8**

USB4k 50%O$_2$Hb Fixed, 15-5 g/dL tHb

Legend:
- 15g/dl
- 10g/dl
- 7g/dl
- 5g/dl

FIG. 9

EP 2 624 754 B1

O2Hb tHb reference
curve matrix

61 rows of O2Hb,
90 to 30%

101 layers, 15 to 5 g/dL

467 columns, 442
nm 539nm

FIG. 10

Spectral Curve Information for Hamamtsu TM 10082 Spectrometer, 90% to 30%

FIG. 11

EP 2 624 754 B1

FIG. 12

FIG. 13

FIG. 14

FIG. 15

FIG. 16

EP 2 624 754 B1

FIG. 17

EP 2 624 754 B1

FIG. 18

EP 2 624 754 B1

FIG. 19

EP 2 624 754 B1

Remitted Spectrum Using Different Integration Times

FIG. 20

EP 2 624 754 B1

**REFERENCES CITED IN THE DESCRIPTION**

**Patent documents cited in the description**

- WO 0009004 A